# EUROPEAN PATENT SPECIFICATION

(11) **EP 3 345 636 B1**
(45) Date of publication and mention of the grant of the patent: **27.05.2020**
(21) Application number: 18158443.4
(22) Date of filing: 10.04.2014
(51) Int. Cl.: A61M 5/00, A61M 5/32, B65B 55/08, B65B 5/02, B65B 7/28

(54) **NEEDLE TIP STORAGE AND REMOVAL DEVICE AND METHODS OF MANUFACTURE THEREOF**
AUFBEWAHRUNGS- UND ENTNAHMEVORRICHTUNG FÜR NADELSPITZEN UND VERFAHREN ZUR HERSTELLUNG DAVON
DISPOSITIF DE RETRAIT ET DE STOCKAGE DE POINTE D'AIGUILLE ET LEURS PROCÉDÉS DE FABRICATION

(30) Priority: 11.04.2013 GB 201306601
(43) Date of publication of application: 11.07.2018
(62) Divisional of application: 14724128.5
(73) Proprietor: OWEN MUMFORD LIMITED, Woodstock, Oxfordshire OX20 1TU (GB)
(72) Inventor: EVANS, Timothy, Oxford, Oxfordshire OX20 1TU (GB); HAWKINS, Alan, Oxford, Oxfordshire OX20 1TU (GB); COWE, Toby, Oxford, Oxfordshire OX20 1TU (GB); VARDE, Andy, Oxford, Oxfordshire OX20 1TU (GB)

(56) References cited:
- EP-A1- 2 522 380
- GB-A- 2 437 923
- US-A- 5 554 129

## Description

This invention relates to needle tip storage and removal devices and to methods of manufacture thereof. Many drugs that need to be injected regularly are delivered by a pen injector or the like (such as our Autopen® injector) which has a removable and disposable needle tip (or "pen tip") which is screwed and unscrewed or otherwise attached and detached by rotary action to the front end of the pen injector to provide a fresh needle for each injection. An example of a typical needle tip is our UniFine® needle tip. This comprises an internally threaded cylindrical hub typically of a plastics material and having a fluted or splined outer cylindrical surface. A needle projects axially forwardly of the hub.

It is known to supply such needle tips in sterile storage containers sealed by means of a foil seal or the like. The storage container has internal splines which engage the external splines on the outer cylindrical surface of the needle tip. The storage container can therefore be used as a spanner or wrench to screw the needle tip into engagement with a threaded portion on the pen injector where this is provided and which can be slid on or off the needle tip. The storage container may provide a similar function in other types of rotary connection. It is preferred that the user, having applied a needle tip to a pen injector and withdrawn the needle tip container to expose the needle tip, should keep the container safe so that it can be used to re-cover the needle. This is so that the container can be used again as a spanner or wrench to unscrew it from the pen injector and then to allow safe disposal of the used needle tip inside the storage container, and also so that the needle is safely covered as the needle tip is being unscrewed.

However, this practice is not always followed with the result that the user may try and remove an unshrouded, used needle tip by hand, with the attendant risk of needle stick injury.

GB2437923 is designed to address this problem and provides a needle tip storage and removal device having opposed, oppositely directed storage and removal compartments, intended to be moulded in one piece in an injection moulding process.

We have found however, this arrangement may be further improved. In particular, in a typical manufacturing facility for such devices, the facility will often be required to switch between insertion, sealing and sterilising a needle tip in a conventional, single container, as well as to be modified to produce runs of needle tips each sealed and sterilised in a storage compartment of a double container of the types shown in GB2437923. This however, requires considerable rejigging of the machinery and can result in a day or so of down time, with the consequent loss in production. In addition, because such devices are normally sterilised by exposure to gamma radiation, and the extent and/or duration of the exposure required is dependant on the mass and volume of material in the device, some of the dosage of radiation is 'wasted' on the integrally formed removal compartment, which does not actually need to be sterilised. Also, because the removal tool needs to be designed to have robust puncture resistance, the integrally formed storage and removal compartments are typically made of a material which is puncture resistant. The puncture resistance material tends to be thicker and so increases the amount of material used which is undesirable for a disposable item.

Accordingly, we have designed a needle tip storage and removal device which allows the materials for the storage compartment to be optimised having regard to the requirements of that compartment, that is (maintaining sterility and ease of sterilisation by radiation and the materials of the removal tool to be optimised for its purpose, typically good puncture resistance and good resilience, economy of production and disposability.

According to the invention, there is provided a needle tip storage and removal device for use with a needle tip comprising a needle hub and a needle extending axially therefrom, which includes:
a storage compartment being sealed by an openable seal element, and
a needle removal housing for receiving a used needle tip, characterised in that
said storage compartment has been formed in a given forming step and contains a needle tip in a sterile environment; and
said removal housing has been formed in a separate forming step, and is connected to or around said storage compartment.

Preferably the hub of said needle tip is generally cylindrical, and said needle tip and said storage compartment are configured to allow said needle tip to be slid into and out of non-rotatable engagement with said removal housing.

Preferably said removal housing is adapted to receive a used needle tip in non-rotatable engagement, when inserted therein.

Preferably said removal housing is configured to receive a needle tip inserted in a direction transverse to said axis.

Preferably said removal housing is adapted to receive a used needle tip when inserted in a direction generally parallel or coincident with said axis.

Preferably said removal housing includes a snap fit engagement arrangement to prevent or resist removal of a used needle tip once inserted into said removal housing.

Preferably said removal housing shrouds the needle of a used needle tip when inserted into said housing, and advantageously said housing fully encloses the needle of said used needle tip.

Preferably the storage container has an outer shape including a larger cylindrical portion and a smaller cylindrical portion interconnected by a bridging portion.

Preferably said removal housing includes at least one ring-shaped portion adapted to surround and secure a portion of the storage container.

Preferably said removal housing includes a spaced ring-shaped portion adapted to surround and secure a further respective spaced portion of said storage compartment.

Preferably each said ring-shaped portion is secured to an adjacent portion of said storage compartment by one or more of: heat welding, ultrasonic welding, adhesive bonding, snap action and mechanical interlock.

Preferably said removal housing includes at least one clip portion adapted to clip around a portion of said storage compartment to secure said removal housing to said storage compartment.

Preferably said removal housing includes two spaced clip portions adapted to clip around respective spaced portions of said storage compartment, to secure said removal housing to said storage compartment.

Preferably said removal housing comprises a larger cylindrical portion merging with a frusto-conical portion which merges with a smaller cylindrical portion.

Preferably said removal housing includes a recess for receiving and locating opposed longitudinal end portions of said storage compartment.

Preferably said removal housing is formed by overmoulding a material over at least a portion of said storage compartment, thereby to provide a removal housing connected around said storage compartment.

Preferably said storage compartment and said removal housing each define respective holding portions for the needle tip such that a needle tip held in the storage compartment is held with its needle extending generally parallel to and facing in the opposite direction to a needle tip held in the removal housing.

In another aspect the invention provides a needle tip storage and removal device for use with a needle tip comprising a cylindrical hub and a needle extending axially therefrom, which comprises a storage compartment formed in a given forming process for containing a needle tip in a sterile environment and for being sealed by an openable seal element, a separately formed outer housing having a recess adapted to receive said storage compartment, and a closure element configured to be moveable between a retracted position in which access to the interior of said storage compartment is allowed, and a closed position in which such access is prevented or inhibited.

Preferably said storage compartment defines an interior recess into which a needle tip may be inserted for non-rotatable engagement, and said outer housing defines a stepped recess having a larger diameter cylindrical portion for receiving said storage compartment and, beyond said larger diameter cylindrical portion, a smaller diameter cylindrical portion into which a needle tip may be inserted for non-rotatable engagement if the larger diameter cylindrical recess is not occupied by a storage compartment.

Preferably said storage compartment includes a removable seal element, and said outer housing and/or said closure element are configured so that the said seal element, if present on said storage compartment, blocks movement of said closure element to its closed position, but when said seal element is absent said closure element may be moved to its closed position.

In another aspect the invention provides a method of producing a needle tip storage and removal device which comprises:
forming a storage compartment in a process step;
forming a removal tool in a separate process step, and
assembling together the storage compartment and the removal tool
wherein the method further includes the step of sterilising the assembly of the needle tip and the storage compartment (10).

Preferably said storage compartment and said removal tool are formed of different materials.

The method may further include the step of inserting a needle tip into said storage compartment and applying a closure element to said compartment hermetically to seal the needle tip in said storage compartment.

The method may further include the step of sterilising the assembly of the needle tip and the storage compartment.

Preferably said step of sterilisation includes irradiation.

Preferably said removal tool comprises a recess for receiving said needle tip.

Preferably said removal tool is joined to said storage compartment by at least one of the following methods:
- ultrasonic welding
- mechanical interlock
- heat welding, and
- adhesion.

Preferably said storage compartment and said removal tool are joined together by forming the removal tool around said storage compartment.

Preferably said removal tool is formed by overmoulding said storage compartment.

Preferably the storage compartment and the removal tool are joined together by forming a removal tool having a recess for receiving the storage compartment and thereafter inserting said storage compartment into said recess.

Whilst the invention has been described above, it extends to any inventive combination or sub-combination of the features set out above, and in the following description, claims or appended drawings.

The invention may be performed in various ways and, by way of example only, various embodiments thereof will now be described in detail, reference being made to the accompanying drawings in which:
Figures 1(a) and (b) are perspective views of a needle tip storage and removal device, with the main components before and after assembly respectively;
Figures 2(a) and (b) are section views of a second embodiment of needle tip storage and removal device before and after assembly respectively;
Figures 3(a) and (b) are section views of a third embodiment of needle tip storage and removal device before and after assembly respectively;
Figures 4(a) and (b) are section views of a fourth embodiment of needle tip storage and removal device before and assembly respectively;
Figures 5(a) to (d) are views of a fifth embodiment of needle tip storage and removal device;
Figures 6(a) to (c) are views of a sixth embodiment of needle tip storage and removal device;
Figures 7(a) to (c) are views of a seventh embodiment of needle tip storage and removal device;
Figures 8(a) to (c) are views of an eighth embodiment of needle tip storage and removal device, prior to insertion of the needle tip storage container;
Figures 9(a) and (b) are views of a ninth embodiment of needle tip storage and removal device in the as supplied and locked conditions respectively;
Figures 10(a) and (b) are exploded section and perspective views respectively of the ninth embodiment of needle tip storage and removal device, and
Figures 11(a) to (j) are successive views showing use of the ninth embodiment of needle tip storage and removal device.

The various embodiments described herein provide needle tip storage and removal devices made up of a storage container or compartment containing a needle tip and hermetically sealed by a removable foil or the like, the needle tip container then being assembled with a removal housing which is or has been formed in a separate forming step. This means that the needle tip storage and removal device may be made by taking a pre-existing needle tip container sealed with the needle tip inside it, and then assembling it with the removal housing. In this way, the moulding of the storage container, loading of the needle tip and subsequent sealing of the container may be optimised for subsequent sterilisation process by gamma irradiation prior to assembly with the removal housing. The sterile needle tip storage container, with needle tip inside, may then be assembled in a sterile manner with the removal housing.

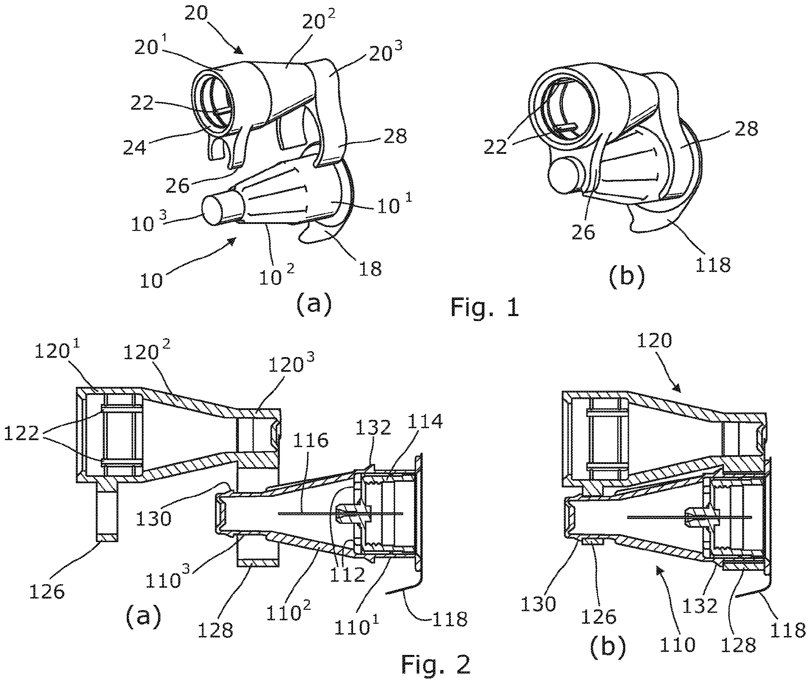

In the embodiments below, the needle tip storage container is made up of a relatively large diameter portion 10¹, which merges with a frustoconical portion 10² which itself merges with a closed tip portion 10³ of relative small diameter, as seen for example in Figures 1(a), 5(a) and 10(b). Internally, the larger cylindrical portion defines an internal cylindrical wall with splines 22 equi-spaced around it, the splines being designed to allow the needle tip hub to be slid into and out of non-rotatable engagement with the needle tip storage container. In this example, the needle tip has a double ended needle (see Figure 2(a)). The needle tip storage container 10 is sealed by a removable foil 18. In the first embodiment, the needle tip removal housing 20 is formed in a separate process and from, typically, different materials but has a similar overall shape and internal shape as that of the needle tip storage container 10. Thus the needle tip removal housing 20 has a relatively large diameter portion 20¹ that merges with a frustoconical portion 20² which itself merges with a cylindrical tip portion 20³. Internally, the large cylindrical portion 20¹ is provided with splines 22 into which a used needle tip may be slid in non-rotatable engagement. The open end of the larger diameter cylindrical portion 20¹ is provided with an internal snap rib 24 designed to lock a used needle tip in the removal housing 20 when it is fully pushed home.

Projecting transversely from the large cylindrical portion 20¹ and the small cylindrical portion 20³ are respective small and large clip portions 26, 28 respectively defining resilient clip recesses which allow the needle tip storage container and the removal housing to be clipped together as shown in Figure 1(b). The clipping action may be permanent or reversible.

Referring now to Figure 2, many features of the second embodiment are similar to those of the first embodiment and will be given similar reference numbers but incremented by 100. In this embodiment, the needle tip removal housing is provided with laterally extending small and large ring portions 126 and 128 respectively. The needle tip storage container is provided with small and large diameter snap rings 130, 132 on the external surface of the small and large diameter cylindrical portions 110³ and 110¹. The needle tip storage container and the needle tip removal housing may be assembled together by disposing them in the position of Figure 2(a) and then bringing them together so that the ribs 130, 132 squeeze through and snap through the ring shaped portions 126 and 128, as seen in Figure 2(b).

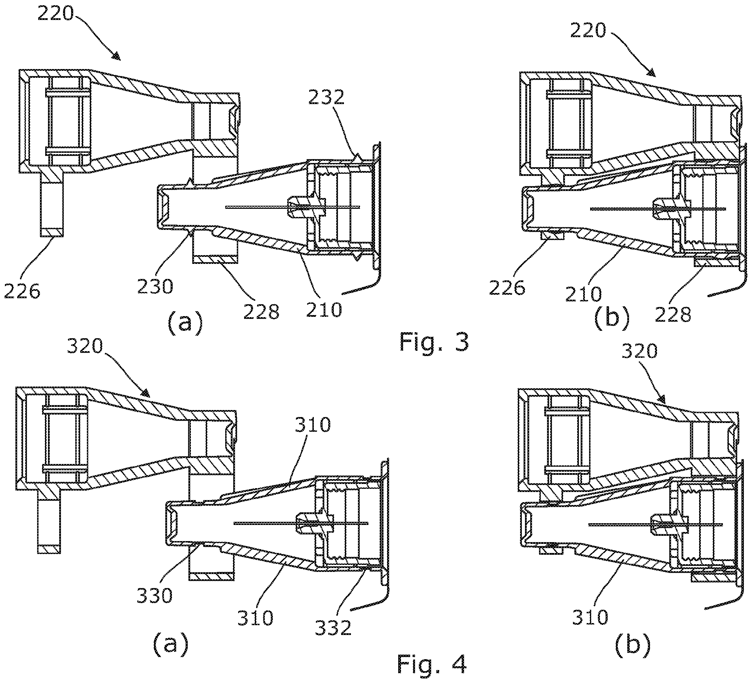

Referring now to Figure 3, many features of the third embodiment are similar to those of the second embodiment and will be given similar reference numbers but incremented by 100. In this embodiment, the small and large diameter portions of the needle tip storage container 210 are formed with annular ribs 230 and 232. The needle tip storage container 210 and the needle tip removal housing 220 are positioned as shown in Figure 3 and then pushed together so that the annular ribs 230 and 232 contact and pass into the inner cylindrical surface of rings 226, 228 on the needle tip removal housing 220. An ultrasonic welding process is then applied to cause the ribs 230 and 232 to fuse or melt with surrounding ring portion 226, 228 permanently to secure the needle tip storage container and the needle tip removal housing together.

Referring now to Figure 4, many features of the fourth embodiment are similar to those of the third embodiment and will be given similar reference numbers but incremented by 100. In this embodiment, the needle tip storage container 310 is provided with annular recesses 330 and 332 in its small and large diameter portions respectively. The needle tip removal housing 320 is identical to that of the fourth embodiment and the needle tip storage container and the needle tip removal housing are assembled together by applying an adhesive to the grooves 330 and 332 and then assembling the two together as shown in Figure 4(b).

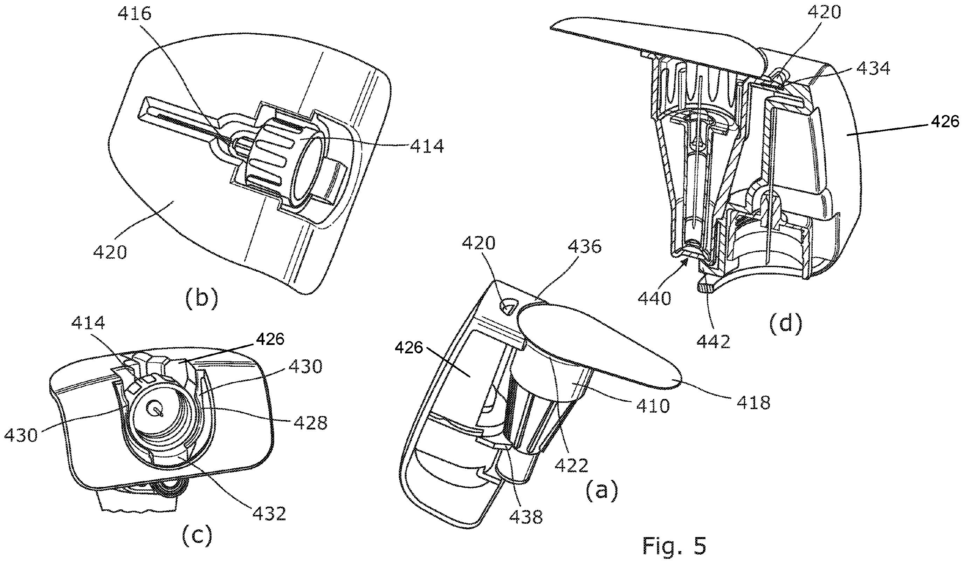

Referring now to Figure 5, in this embodiment a needle tip storage container 410 similar to that of the previous embodiment, containing a needle tip of the type described above and sealed by a foil 418 is provided with a tab 420 that extends transversely from a flange 422 provided at the open end of the larger cylindrical portion of the needle tip storage container. The flange 422 provides a planar radial surface to which the foil 418 is heat-sealed. The tab 420 is formed integrally in the same moulding process as the main portion of the needle tip storage container 410.

In this embodiment, the needle tip removal housing is based on our existing UniGuard® device, as described in WO 2005/102424. The needle tip removal housing 426 has an opening 428 of outline generally matching and adapted to receive the hub 414 and needle 416 of a needle tip when inserted laterally. The opening includes a U-shaped cradle portion 428 which has two abutments 430 past which the needle hub snaps when inserted laterally. Once in its fully home position, the needle hub engages splines (not shown) on the interior of the U-shaped portion 428 to engage it non-rotatably. If required, there may be a two stage engagement action whereby the user initially inserts the needle hub laterally and then pushes it axially (in the direction of the needle) to a fully home position. In this latter position, the needle hub may snap past a tongue 432 to hold it against reverse axial movement. As seen particularly in Figures 5(a) and5(d), the needle removal housing 426 is provided with a recess 434 into which the tab 420 on the flange of the needle tip storage container be snapped irreversibly. Snapping the tab 420 into the recess 434 holds the needle tip storage container closely against the underside of the needle tip removal housing 426, with portions thereof cradled by supports 436, 438 and the smaller diameter end of the needle housing is provided with a dished portion 440 over into the edge of which hooks a protrusion 442 to hold the needle tip storage container and the needle tip removal housing securely together.

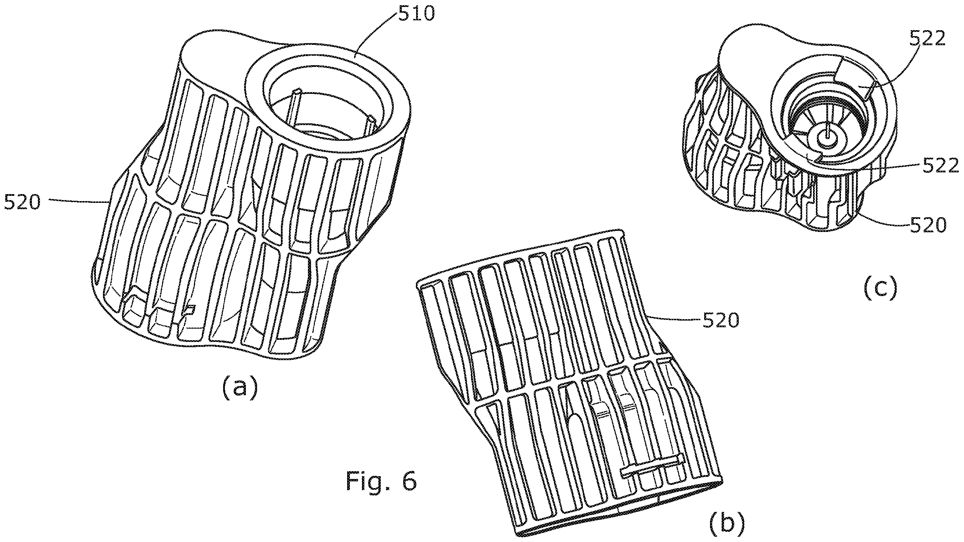

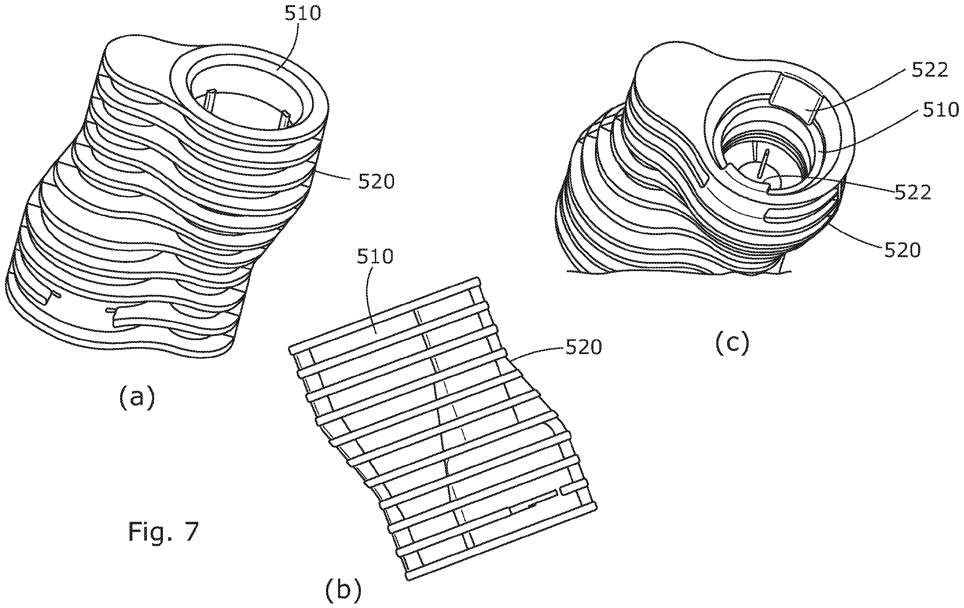

Referring now to the embodiments of Figures 6 and 7, here a two-shot or overmoulding process is used to initially form a needle tip storage container 510 of similar form to those of the earlier embodiments which can then be fitted with a needle tip sealed and then sterilised. In the second moulding step, using a different plastics material, a needle tip removal housing 520 is moulded which has a main needle tip receiving portion with an integral frame structure embracing and connecting the needle tip storage container. The needle tip removal portion may include flexible tabs 522 to prevent removal of a used needle tip once inserted into the needle tip removal portion. As with the other embodiments, the needle tip removal portion has a recess configured non-rotatably to receive a used needle tip. In Figure 6, the main frame elements encasing the needle tip storage container run generally longitudinally, whereas in the arrangement of Figure 7, they run generally circumferentially.

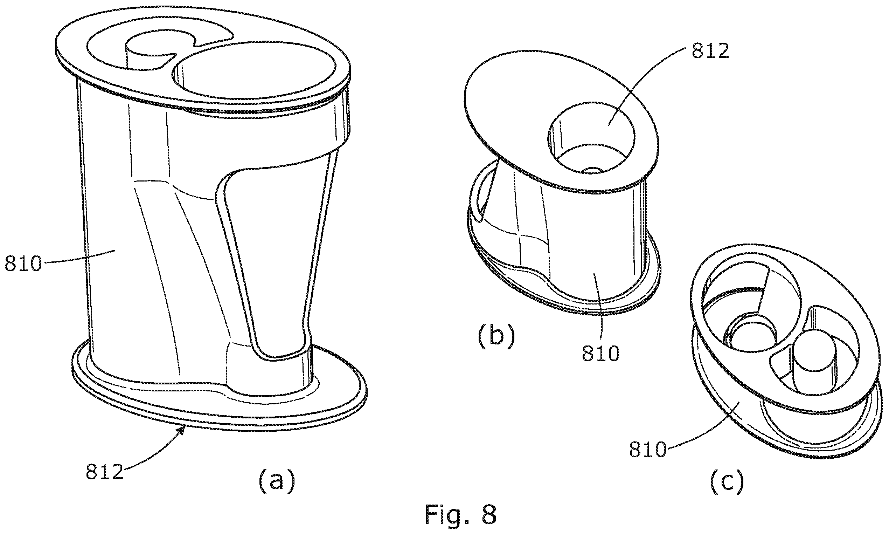

Referring now to Figure 8, in this embodiment a needle tip removal housing 810 is provided with a needle tip removal portion 812 designed non-rotatably to receive a used needle tip as before. Adjacent to the needle tip removal portion there is a shaped recess 814 designed to receive by a snap fit a needle tip storage container. As previously, therefore, each of the needle tip removal housing and a needle tip storage container may be separately formed and then assembled together to form the final product.

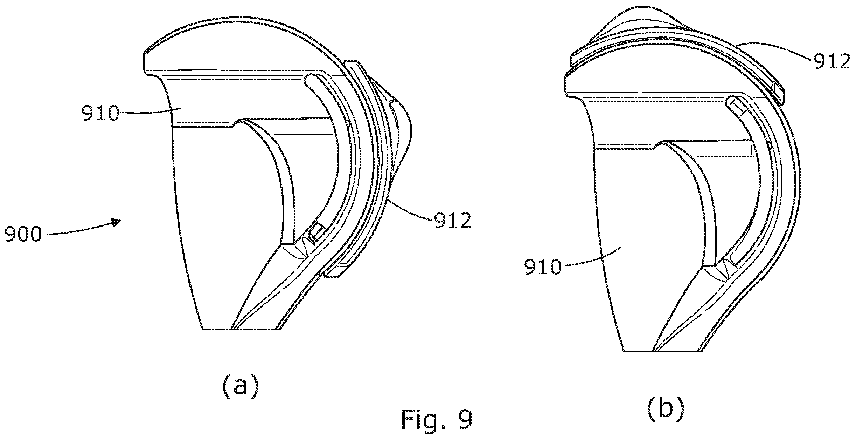

Referring now to the embodiment illustrated in Figures 9 to 11, here the needle tip storage and removal device is made up by assembling a needle tip within the needle tip storage container to provide sterile primary packaging as previously, with that assembly then being fitted into a disposal system 900, thereby allowing broader material selection for the disposal system and reducing processing costs. The needle disposal system comprises a housing 910 with a moveable 912 lid that can be moved from an open position, in which access to the needle tip storage container is allowed, and a closed position in which access to the needle storage container is obstructed. The device may use any suitable form of closing movement but, in the embodiment of Figures 9 to 11, an arcuate sliding movement is used to slide a cover from the open position shown in Figure 9(a) to the closed position shown in Figure 9(b). The cover includes a latching mechanism 914, 916 to latch it in its closed position.

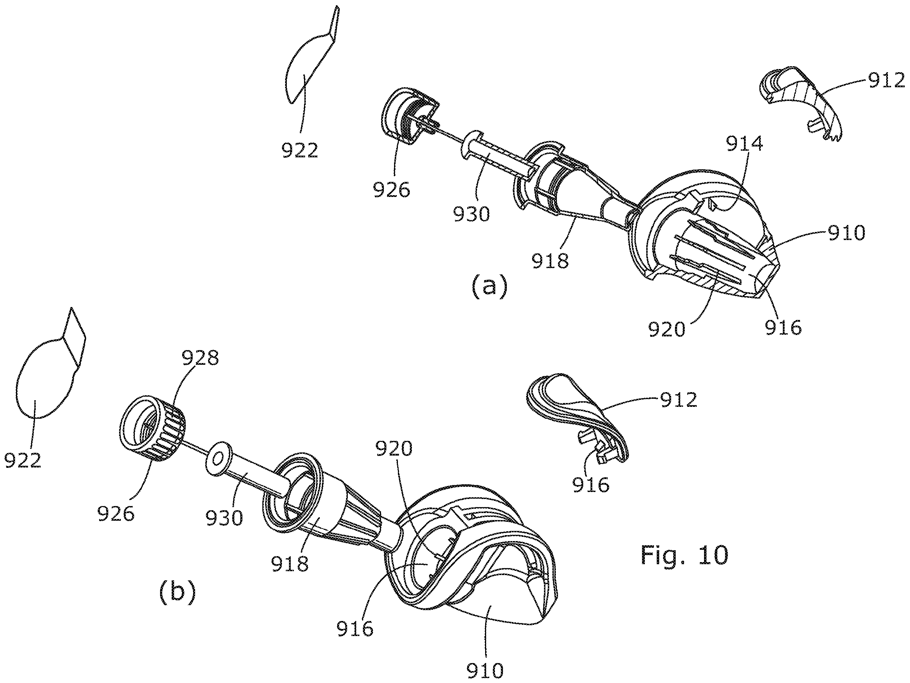

Referring now to Figure 10, the housing 910 has a recess 916 designed to receive the needle tip storage container 918. The needle tip storage container may be an interference fit within the recess or it may snap into place. As seen more particularly in Figure 10(a), the recess is provided with stepped internal radial ribs 920 which co-operate with formations 922 on the outside of the needle tip storage container to prevent rotation there between. The latching mechanism for locking the cover 912 in its closed position comprises a tooth 914 on the housing which co-operates with the corresponding flexible tooth on the cover 916. The device of Figure 10 is designed so that, when a needle storage container 918 is fitted into the recess, the foil 922 on the needle storage container 918 obstructs movement of the cover 912 towards its closed position, so that the cover can only be slid closed once the foil has been removed.

Referring again to Figure 10(a), the radial ribs 920 are stepped inwardly and designed so that, in the event that the needle tip storage container should somehow become dislodged from the disposal system, as a failsafe arrangement the recess can slideably receive a non-rotatable fashion the needle hub 926, with the inwardly stepped ribs 920-co-operating with the splines 928 on the hub.

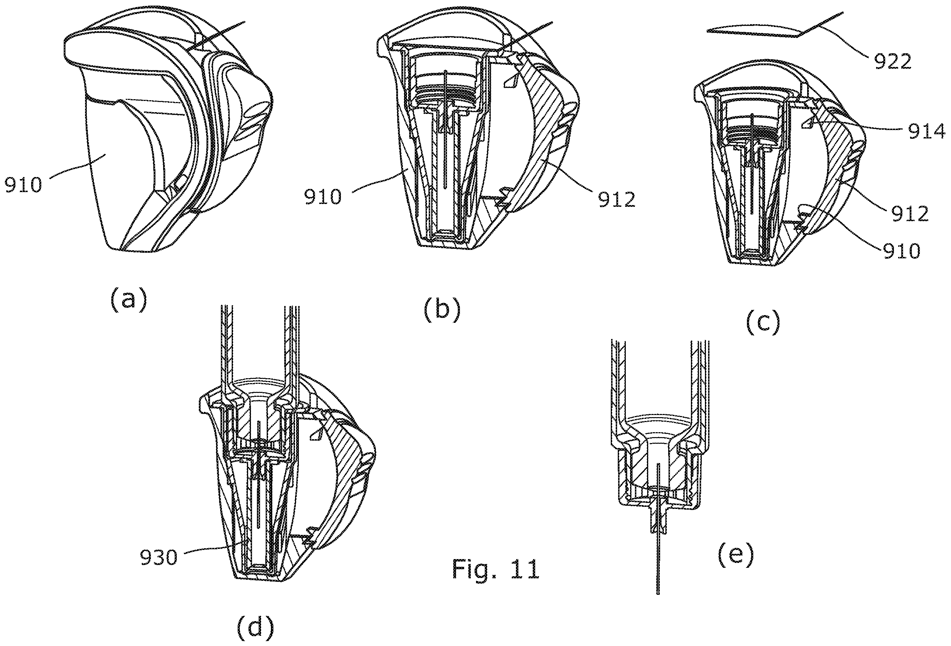

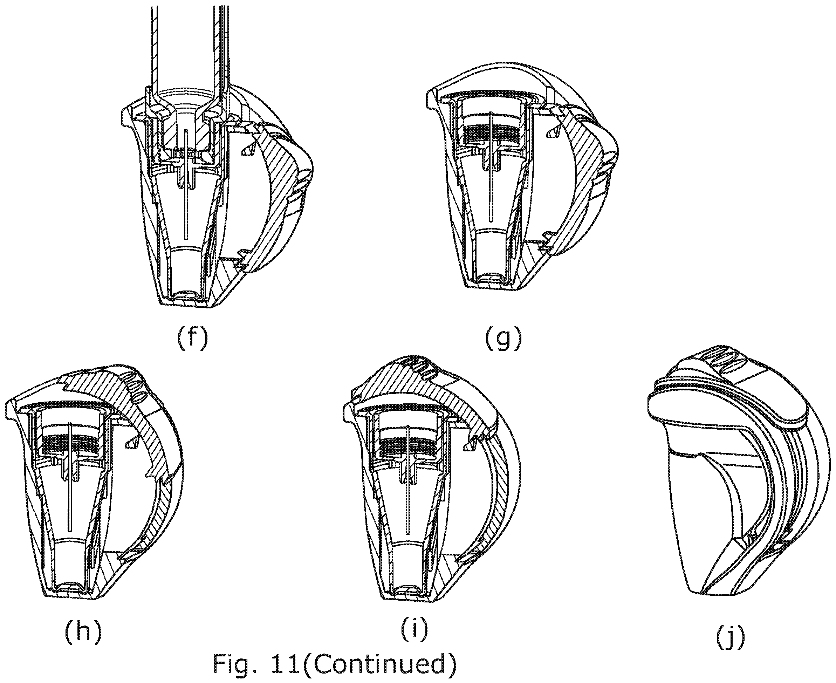

Referring to Figure 11, when the device is as supplied, the cover 912 is its open position and prevented from moving to the closed position by the tab of the foil seal 922. The user prepares the device by removing the foil seal 922 and then inserting a pen and screwing it on to the needle to collect the needle. The user then withdraws the pen from the device and then removes an inner needle shield 930 to expose the needle ready for an injection (Figure 11(e)). After the injection, the user replaces the needle hub into the needle storage container and unscrews the pen. This can be seen in Figure 11(f). As an additional feature, should the primary packaging have fallen out, the pen can be inserted deeper into the disposal system to engage the needle hub with the radial splines. Having removed the pen, the cover may be slid around the body to cover the needle with the tooth on the cover snapping past the tooth on the housing to lock it in place. The used needle is then in a safe condition ready for disposal.

## Claims

1. A needle tip storage and removal device for use with an injection needle tip comprising a needle hub and a needle extending axially therefrom, which includes:
a storage compartment (10) being sealed by an openable seal element (18), and
a needle removal housing (20) for receiving a used needle tip, **characterised in that**
said storage compartment (10) has been formed in a given forming step and contains a needle tip in a sterile environment; and
said removal housing (20) has been formed in a separate forming step, and is connected to or around said storage compartment (10) by a connecting means, wherein said removal housing (20) and said storage compartment (10) are separate parts.

2. A needle tip storage and removal device according to Claim 1, wherein the hub of said needle tip comprises a splined outer cylindrical surface and said removal housing (20) comprises a large cylindrical portion (20¹) provided with internal splines (22) such that it is adapted to receive a used needle tip in non-rotatable engagement, when inserted therein.

3. A needle tip storage and removal device according to Claim 2, wherein said removal housing (20) is configured to receive a needle tip inserted in a direction transverse to the needle axis or wherein said removal housing (20) is adapted to receive a used needle tip when inserted in a direction generally parallel or coincident with said needle axis.

4. A needle tip storage and removal device according to any of the preceding Claims, wherein said removal housing (20) shrouds the needle of a used needle tip when inserted into said housing (20) and optionally wherein said housing (20) fully encloses the needle of said used needle tip.

5. A needle tip storage and removal device according to any of the preceding Claims, wherein the storage container (10) has an outer shape including a larger cylindrical portion (10¹) and a smaller cylindrical portion (10³) interconnected by a bridging portion (10²) and/or wherein the hub of said needle tip is generally cylindrical having a splined outer surface and said larger cylindrical portion (10¹) of the storage compartment (10) defines an internal cylindrical wall with splines (12), such that said needle tip and said storage compartment (10) are configured to allow said needle tip to be slid into and out of non-rotatable engagement with said storage compartment (10) and/or wherein said removal housing includes a snap fit engagement arrangement to prevent or resist removal of a used needle tip once inserted into said removal housing.

6. A needle tip storage and removal device according to any of the preceding Claims, wherein said removal housing (20) includes at least one ring-shaped portion (26) adapted to surround and secure a portion of the storage container (10) and optionally wherein said removal housing (20)_includes a spaced ring-shaped portion (28) adapted to surround and secure a further respective spaced portion of said storage compartment (10).

7. A needle tip storage and removal device according to Claim 6, wherein each said ring-shaped portion (26, 28) is secured to an adjacent portion of said storage compartment (10) by one or more of: heat welding, ultrasonic welding, adhesive bonding, snap action and mechanical interlock.

8. A needle tip storage and removal device according to any of Claims 1 to 5, wherein said removal housing (20) includes at least one clip portion adapted to clip around a portion of said storage compartment (10) to secure said removal housing (20) to said storage compartment (10) and optionally wherein said removal housing (20) includes two spaced clip portions (26, 28) adapted to clip around respective spaced portions of said storage compartment (10), to secure said removal housing (20) to said storage compartment (10).

9. A needle tip storage and removal device according to any of the preceding Claims, wherein said removal housing (20) comprises a larger cylindrical portion (20¹) merging with a frusto-conical portion (20²) which merges with a smaller cylindrical portion (20³) and/or wherein said removal housing (20) includes a recess (434) for receiving and locating opposed longitudinal end portions of said storage compartment (10).

10. A needle tip storage and removal device according to any of the preceding Claims, wherein said removal housing (20) is formed by overmoulding a material over at least a portion of said storage compartment (10), thereby to provide a removal housing (20) connected around said storage compartment (10) and/or wherein said storage compartment (10) and said removal housing (20) each define respective holding portions for the needle tip such that a needle tip held in the storage compartment (10) is held with its needle extending generally parallel to and facing in the opposite direction to a needle tip held in the removal housing (20).

11. A method of producing a needle tip storage and removal device according to any of claims 1-10, the method comprising:
forming a storage compartment (10) in a process step;
forming a removal tool (20) in a separate process step, and
assembling together the storage compartment (10) and the removal tool (20)
wherein the method further includes the step of sterilising the assembly of the needle tip and the storage compartment (10).

12. A method according to Claim 11, wherein said storage compartment (10) and said removal tool (20) are formed of different materials.

13. A method according to Claim 11 or Claim 12, which includes the step of inserting a needle tip into said storage compartment (10) and applying a closure element (18) to said compartment (10) hermetically to seal the needle tip in said storage compartment (10) and optionally which further includes the step of sterilising the assembly of the needle tip and the storage compartment (10) and optionally wherein said step of sterilisation includes irradiation.

14. A method according to any of Claims 11 to 13, wherein said removal tool (20) comprises a recess (434) for receiving said needle tip and/or wherein said removal tool (20) is joined to said storage compartment (10) by at least one of the following methods:
• ultrasonic welding
• mechanical interlock
• heat welding, and
• adhesion.

15. A method according to any of Claims 11 to 14, wherein said storage compartment (10) and said removal tool (20) are joined together by forming the removal tool (20) around said storage compartment (10) and optionally wherein said removal tool (20) is formed by overmoulding said storage compartment (10).

## Patentansprüche

1. Nadelspitzen-Aufbewahrungs- und Entnahmevorrichtung zur Verwendung mit einer Injektionsnadelspitze, umfassend eine Nadelnabe und eine Nadel, die sich axial davon erstreckt, Folgendes enthaltend:
ein Aufbewahrungsfach (10), das durch ein zu öffnendes Dichtungselement (18) abgedichtet ist, und
ein Nadelbeseitigungsgehäuse (20) zum Aufnehmen einer gebrauchten Nadelspitze, **dadurch gekennzeichnet, dass**
das Aufbewahrungsfach (10) in einem festgelegten Umformschritt gebildet wurde und eine Nadelspitze in einer sterilen Umgebung enthält; und
das Beseitigungsgehäuse (20) in einem separaten Umformschritt geformt wurde und durch Verbindungsmittel mit dem oder um das Aufbewahrungsfach (10) verbunden ist, wobei das Beseitigungsgehäuse (20) und das Aufbewahrungsfach (10) separate Teile sind.

2. Nadelspitzen-Aufbewahrungs- und Entnahmevorrichtung nach Anspruch 1, wobei die Nabe der Nadelspitze eine genutete äußere zylindrische Oberfläche und das Beseitigungsgehäuse (20) einen großen zylindrischen Abschnitt (20¹) umfasst, der mit inneren Rippen (22) versehen ist, sodass er geeignet ist, eine gebrauchte Nadelspitze in nichtdrehbarem Eingriff aufzunehmen, wenn sie darin eingeführt wird.

3. Nadelspitzen-Aufbewahrungs- und Entnahmevorrichtung nach Anspruch 2, wobei das Beseitigungsgehäuse (20) dazu konfiguriert ist, eine Nadelspitze aufzunehmen, die in einer Richtung quer zu der Nadelachse eingesetzt wird, oder wobei das Beseitigungsgehäuse (20) geeignet ist, eine gebrauchte Nadelspitze aufzunehmen, wenn sie in einer Richtung eingeführt wird, die im Allgemeinen parallel zu der Nadelachse ist oder mit dieser zusammenfällt.

4. Nadelspitzen-Aufbewahrungs- und Entnahmevorrichtung nach einem der vorangehenden Ansprüche, wobei das Beseitigungsgehäuse (20) die Nadel einer gebrauchten Nadelspitze abschirmt, wenn sie in das Gehäuse (20) eingesetzt ist, und wobei gegebenenfalls das Gehäuse (20) die Nadel der gebrauchten Nadelspitze vollständig umschließt.

5. Nadelspitzen-Aufbewahrungs- und Entnahmevorrichtung nach einem der vorangehenden Ansprüche, wobei der Aufbewahrungsbehälter (10) eine äußere Form mit einem größeren zylindrischen Abschnitt (10¹) und einem kleineren zylindrischen Abschnitt (10³) aufweist, die durch einen Überbrückungsabschnitt (10²) verbunden sind, und/oder wobei die Nabe der Nadelspitze im Allgemeinen zylindrisch ist und eine genutete Außenfläche aufweist und wobei der größere zylindrische Abschnitt (10¹) des Aufbewahrungsfaches (10) eine zylindrische Innenwand mit Rippen (12) definiert, sodass die Nadelspitze und das Aufbewahrungsfach (10) so konfiguriert sind, dass die Nadelspitze in einen nicht drehbaren Eingriff mit dem Aufbewahrungsfach (10) ein- und aus diesem austreten kann, und/oder wobei das Beseitigungsgehäuse eine Schnappverschluss-Eingriffanordnung aufweist, um das Entfernen einer gebrauchten Nadelspitze, sobald diese in das Beseitigungsgehäuse eingeführt wurde, zu verhindern bzw. diesem zu widerstehen.

6. Nadelspitzen-Aufbewahrungs- und Entnahmevorrichtung nach einem der vorhergehenden Ansprüche, wobei das Beseitigungsgehäuse (20) zumindest einen ringförmigen Abschnitt (26) beinhaltet, der dazu geeignet ist, einen Abschnitt des Aufbewahrungsfaches (10) zu umgeben und zu sichern, und wobei das Beseitigungsgehäuse (20) einen beabstandeten ringförmigen Abschnitt (28) beinhaltet, der dazu geeignet ist, einen weiteren entsprechenden beabstandeten Abschnitt des Aufbewahrungsfaches (10) zu umgeben und zu sichern.

7. Nadelspitzen-Aufbewahrungs- und Entnahmevorrichtung nach Anspruch 6, wobei jeder ringförmige Abschnitt (26, 28) durch eines oder mehrere der folgenden Verfahren an einem benachbarten Abschnitt des Aufbewahrungsfaches (10) befestigt ist: Heißschweißen, Ultraschallschweißen, Kleben, Schnappverschluss und mechanische Verriegelung.

8. Nadelspitzen-Aufbewahrungs- und Entnahmevorrichtung nach einem der Ansprüche 1 bis 5, wobei das Beseitigungsgehäuse (20) mindestens einen Klammerabschnitt umfasst, der zum Festklemmen um einen Abschnitt des Aufbewahrungsfachs (10) herum geeignet ist, um das Beseitigungsgehäuse (20) an dem Aufbewahrungsfach (10) zu befestigen, und wobei gegebenenfalls das Beseitigungsgehäuse (20) zwei beabstandete Klammerabschnitte (26, 28) enthält, die zum Festklemmen um entsprechend beabstandete Abschnitte des Aufbewahrungsfachs (10) geeignet sind, um das Beseitigungsgehäuse (20) an dem Aufbewahrungsfach (10) zu befestigen.

9. Nadelspitzen-Aufbewahrungs- und Entnahmevorrichtung nach einem der vorhergehenden Ansprüche, wobei das Beseitigungsgehäuse (20) einen mit einem kegelstumpfförmigen Abschnitt (20²) verschmelzenden größeren zylindrischen Abschnitt (20¹) aufweist, der mit einem kleineren zylindrischen Abschnitt (20³) verschmilzt, und/oder wobei das Beseitigungsgehäuse (20) eine Aussparung (434) zum Aufnehmen und Lokalisieren gegenüberliegender länglicher Endabschnitte des Aufbewahrungsfachs (10) enthält.

10. Nadelspitzen-Aufbewahrungs- und Entnahmevorrichtung nach einem der vorhergehenden Ansprüche, wobei das Beseitigungsgehäuse (20) durch Aufformen eines Materials über zumindest einen Teil des Aufbewahrungsfachs (10) gebildet wird, um derart ein um das Aufbewahrungsfach (10) verbundenes Beseitigungsgehäuse (20) bereitzustellen, und/oder wobei das Aufbewahrungsfach (10) und das Beseitigungsgehäuse (20) jeweils entsprechende Halteabschnitte für die Nadelspitze definieren, sodass eine in dem Aufbewahrungsfach (10) gehaltene Nadelspitze so gehalten wird, dass sich ihre Nadel im Allgemeinen parallel zu einer in dem Beseitigungsgehäuse (20) gehaltenen Nadelspitze und in eine diesem entgegengesetzte Richtung weisend erstreckt.

11. Verfahren zum Herstellen einer Nadelspitzen-Aufbewahrungs- und Entnahmevorrichtung nach einem der Ansprüche 1-10, wobei das Verfahren umfasst:
Bilden eines Aufbewahrungsfachs (10) in einem Prozessschritt;
Bilden eines Beseitigungswerkzeugs (20) in einem separaten Prozessschritt, und
Zusammenbauen des Aufbewahrungsfachs (10) und des Beseitigungswerkzeugs (20),
wobei das Verfahren ferner den Schritt des Sterilisierens der Anordnung der Nadelspitze und des Aufbewahrungsfachs (10) umfasst.

12. Verfahren nach Anspruch 11, wobei das Aufbewahrungsfach (10) und das Beseitigungswerkzeug (20) aus unterschiedlichen Materialien gebildet sind.

13. Verfahren nach Anspruch 11 oder Anspruch 12, das den Schritt des Einführens einer Nadelspitze in das Aufbewahrungsfach (10) und das Anlegen eines Verschlusselements (18) an dem Fach (10) zum hermetischen Versiegeln der Nadelspitze in dem Aufbewahrungsfach (10) umfasst und gegebenenfalls ferner den Schritt des Sterilisierens der Anordnung der Nadelspitze und des Aufbewahrungsfachs (10) umfasst und wobei der Schritt des Sterilisierens gegebenenfalls eine Bestrahlung umfasst.

14. Verfahren nach einem der Ansprüche 11 bis 13, wobei das Beseitigungswerkzeug (20) eine Aussparung (434) zum Aufnehmen der Nadelspitze umfasst und/oder wobei das Beseitigungswerkzeug (20) mit dem Aufbewahrungsfach (10) durch mindestens eine der folgenden Methoden verbunden ist:
• Ultraschallschweißen
• mechanische Verriegelung
• Heißschweißen, und
• Kleben.

15. Verfahren nach einem der Ansprüche 11 bis 14, wobei das Aufbewahrungsfach (10) und das Beseitigungswerkzeug (20) miteinander verbunden werden, indem das Beseitigungswerkzeug (20) um das Aufbewahrungsfach (10) gebildet wird und wobei gegebenenfalls das Beseitigungswerkzeug (20) durch Überformen des Aufbewahrungsfachs (10) gebildet wird.

## Revendications

1. Dispositif de stockage et de retrait de pointe d'aiguille à utiliser avec une pointe d'aiguille d'injection comprenant un moyeu d'aiguille et une aiguille s'étendant axialement à partir de celui-ci, qui inclut :
un compartiment de stockage (10) étant scellé par un élément d'étanchéité ouvrable (18), et
un boîtier de retrait d'aiguille (20) pour recevoir une pointe d'aiguille usagée, **caractérisé en ce que**
ledit compartiment de stockage (10) a été formé au cours d'une étape de formation donnée et contient une pointe d'aiguille dans un environnement stérile ; et
ledit boîtier de retrait (20) a été formé dans une étape de formation séparée, et est connecté au ou autour dudit compartiment de stockage (10) par un moyen de connexion, dans lequel ledit boîtier de retrait (20) et ledit compartiment de stockage (10) sont des parties distinctes.

2. Dispositif de stockage et de retrait de pointe d'aiguille selon la revendication 1, dans lequel le moyeu de ladite pointe d'aiguille comprend une surface cylindrique externe cannelée et ledit boîtier de retrait (20) comprend une grande partie cylindrique (20¹) pourvue de cannelures internes (22) de telle sorte qu'il est adapté pour recevoir une pointe d'aiguille usagée en engagement non rotatif, lorsqu'elle y est insérée.

3. Dispositif de stockage et de retrait de pointe d'aiguille selon la revendication 2, dans lequel ledit boîtier de retrait (20) est configuré pour recevoir une pointe d'aiguille insérée dans une direction transversale à l'axe de l'aiguille ou dans lequel ledit boîtier de retrait (20) est adapté pour recevoir une pointe d'aiguille usagée lorsqu'elle est insérée dans une direction généralement parallèle ou coïncidant avec ledit axe de l'aiguille.

4. Dispositif de stockage et de retrait de pointe d'aiguille selon l'une quelconque des revendications précédentes, dans lequel ledit boîtier de retrait (20) enveloppe l'aiguille d'une pointe d'aiguille usagée lorsqu'elle est insérée dans ledit boîtier (20) et éventuellement dans lequel ledit boîtier (20) enferme entièrement l'aiguille de ladite pointe d'aiguille usagée.

5. Dispositif de stockage et de retrait de pointe d'aiguille selon l'une quelconque des revendications précédentes, dans lequel le compartiment de stockage (10) a une forme externe incluant une plus grande partie cylindrique (10¹) et une plus petite partie cylindrique (10³) interconnectées par une partie de pontage (10²) et/ou dans lequel le moyeu de ladite pointe d'aiguille est généralement cylindrique ayant une surface externe cannelée et ladite plus grande partie cylindrique (10¹) du compartiment de stockage (10) définit une paroi cylindrique interne avec des cannelures (12), de telle sorte que ladite pointe d'aiguille et ledit compartiment de stockage (10) sont configurés pour permettre à ladite pointe d'aiguille d'être glissée dans et hors de l'engagement non rotatif avec ledit compartiment de stockage (10) et/ou dans lequel ledit boîtier de retrait inclut un agencement d'engagement par encliquetage pour empêcher ou résister au retrait d'une pointe d'aiguille usagée une fois insérée dans ledit boîtier de retrait.

6. Dispositif de stockage et de retrait de pointe d'aiguille selon l'une quelconque des revendications précédentes, dans lequel ledit boîtier de retrait (20) inclut au moins une partie en forme d'anneau (26) adaptée pour entourer et fixer une partie du compartiment de stockage (10) et éventuellement dans lequel ledit boîtier de retrait (20)_comprend une partie en forme d'anneau espacée (28) adaptée pour entourer et fixer une autre partie espacée respective dudit compartiment de stockage (10).

7. Dispositif de stockage et de retrait de pointe d'aiguille selon la revendication 6, dans lequel chacune desdites parties en forme d'anneau (26, 28) est fixée à une partie adjacente dudit compartiment de stockage (10) par un ou plusieurs des éléments suivants : soudage à chaud, soudage à ultrasons, collage, encliquetage et verrouillage mécanique.

8. Dispositif de stockage et de retrait de pointe d'aiguille selon l'une quelconque des revendications 1 à 5, dans lequel ledit boîtier de retrait (20) inclut au moins une partie d'attache adaptée pour s'attacher autour d'une partie dudit compartiment de stockage (10) pour fixer ledit boîtier de retrait (20) audit compartiment de stockage (10) et éventuellement dans lequel ledit boîtier de retrait (20) inclut deux parties d'attache espacées (26, 28) adaptées pour s'attacher autour des parties espacées respectives dudit compartiment de stockage (10), pour fixer ledit boîtier de retrait (20) sur ledit compartiment de stockage (10).

9. Dispositif de stockage et de retrait de pointe d'aiguille selon l'une quelconque des revendications précédentes, dans lequel ledit boîtier de retrait (20) comprend une plus grande partie cylindrique (20¹) fusionnant avec une partie tronconique (20²) qui fusionne avec une plus petite partie cylindrique (20³) et/ou dans lequel ledit boîtier de retrait (20) inclut un évidement (434) pour recevoir et localiser des parties d'extrémité longitudinales opposées dudit compartiment de stockage (10).

10. Dispositif de stockage et de retrait de pointe d'aiguille selon l'une quelconque des revendications précédentes, dans lequel ledit boîtier de retrait (20) est formé en surmoulant un matériau sur au moins une partie dudit compartiment de stockage (10), pour ainsi fournir un boîtier de retrait (20) connecté autour dudit compartiment de stockage (10) et/ou dans lequel ledit compartiment de stockage (10) et ledit boîtier de retrait (20) définissent chacun des parties de maintien respectives pour la pointe d'aiguille de telle sorte qu'une pointe d'aiguille maintenue dans le compartiment de stockage (10) est maintenue avec son aiguille s'étendant généralement parallèlement à et faisant face dans la direction opposée à une pointe d'aiguille maintenue dans le boîtier de retrait (20).

11. Procédé de production d'un dispositif de stockage et de retrait de pointe d'aiguille selon l'une quelconque des revendications 1-10, le procédé comprenant :
la formation d'un compartiment de stockage (10) dans une étape de traitement ;
la formation d'un outil de retrait (20) dans une étape de traitement séparée, et
l'assemblage du compartiment de stockage (10) et de l'outil de retrait (20)
dans lequel le procédé inclut en outre l'étape de stérilisation de l'assemblage de la pointe de l'aiguille et du compartiment de stockage (10).

12. Procédé selon la revendication 11, dans lequel ledit compartiment de stockage (10) et ledit outil de retrait (20) sont formés de matériaux différents.

13. Procédé selon la revendication 11 ou la revendication 12, qui inclut l'étape d'insertion d'une pointe d'aiguille dans ledit compartiment de stockage (10) et d'application d'un élément de fermeture (18) audit compartiment (10) pour sceller hermétiquement la pointe d'aiguille dans ledit compartiment de stockage (10) et éventuellement qui inclut en outre l'étape de stérilisation de l'assemblage de la pointe de l'aiguille et du compartiment de stockage (10) et éventuellement dans lequel ladite étape de stérilisation inclut l'irradiation.

14. Procédé selon l'une quelconque des revendications 11 à 13, dans lequel ledit outil de retrait (20) comprend un évidement (434) pour recevoir ladite pointe d'aiguille et/ou dans lequel ledit outil de retrait (20) est joint audit compartiment de stockage (10) par au moins l'une des méthodes suivantes :
• soudage à ultrasons
• verrouillage mécanique
• soudage à chaud, et
• collage.

15. Procédé selon l'une quelconque des revendications 11 à 14, dans lequel ledit compartiment de stockage (10) et ledit outil de retrait (20) sont réunis en formant l'outil de retrait (20) autour dudit compartiment de stockage (10) et éventuellement dans lequel ledit l'outil de retrait (20) est formé par surmoulage dudit compartiment de stockage (10).
